# EUROPEAN PATENT SPECIFICATION

(11) **EP 1 189 653 B1**
(45) Date of publication and mention of the grant of the patent: **08.03.2006**
(21) Application number: 99926110.0
(22) Date of filing: 01.06.1999
(51) Int. Cl.: A61M 25/00, A61M 39/22

(54) **Medical connector**
Medizinischer Konnektor
Raccord médical

(43) Date of publication of application: 27.03.2002
(73) Proprietor: Creative Plastic Technology, LLC, Upland, CA 91786 (US)
(72) Inventor: BONALDO, Jean, M., Upland, CA 91786 (US)
(74) Representative: Ruschke, Hans Edvard
(86) International application number: PCT/US1999/012141
(87) International publication number: WO 2001/023026

(56) References cited:
- US-A- 3 626 980
- US-A- 3 794 042
- US-A- 5 045 068
- US-A- 5 836 924
- US-A- 5 947 954

## Description

### Background of the Invention and Prior Art

The present invention relates to medical connectors for blood transfer, intravenous fluid supply, medication dosage and the like.

United States Patents Nos. 5,273,533 of December 28, 1993 and 5,306,243 of April 26, 1994 each issued to Bonaldo disclose a medical connector valve which employs an elastomeric valve element in the form of an elastomeric septum or fluid barrier disposed in a two part plastic housing. The septum is pierced by an upstream pointed cannula to make the fluid connection. Disconnection of the flow line allows the elastomer to re-seal the connector. Valve opening and closing is regulated by rotating a fluid line connection with respect to a housing in which the cannula and septum are mounted. Such connectors are relatively expensive due to the presence of a cannula and the mounting thereof and are increasingly more likely to leak or become contaminated with particulate material of the septum due to repeated use.

US Patent 5,045,068 which is used as a basis for the preamble of claim 1, shows a flow rate regulator including a first cylindrical member, a second cylindrical member and a disc-shaped member to regulate the flow rate of liquid flowing through a transfusion unit. The liquid is introduced into the second cylindrical member via an inlet port. It flows through the disc-shaped member via a through hole and flows further along a peripherally extending groove in the first cylindrical member. Then it is discharged from the first cylindrical member via an outlet port after the flow rate is regulated as required. The disc-shaped member is fitted in the second cylindrical member so as not to be rotated relative to the latter, and the subassembly is then fitted into the first cylindrical member. The peripherally extending groove is so designed that its cross-sectional area is gradually varied on the bottom portion of the first cylindrical member. The through hole in the disc-shaped member is communicated with the peripherally extending groove. A position where the through hole in the disc-shaped member is communicated with the peripherally extending groove in the first cylindrical member is varied by rotating the first cylindrical member so that a cross-sectional area of the peripherally extending groove is varied correspondingly. This is a flow rate regulator in which the amount of flow is regulated by the positioning of the parts so that flow passes through a cross-sectional area of the V-shaped groove of selected size. The groove collects fluid and sediment whenever the regulator is not open.

Medical connectors frequently must be repeatedly opened and closed during patient care. In hospital environments such as intensive care units medical connectors may be actuated or cycled many times and must remain leak free so as to safely avoid introduction of contaminants such as cotton fibers from swabs used to clean the connectors and inadvertent introduction of air bubbles which cause embolisms and patient death.

Medical connectors which use resilient flow barriers which are repeatedly pierced during use of the connector become more subject to fluid leakage with increased actuation cycles, particularly if connected in an infusion pump line which may subject the connector to pressures as high as 1.72 bar (27 psi).

### Objects and Features of the Invention

It is the primary objective of the invention to provide a medical connector which remains leak and particle free despite repetitive use and which does not employ a resilient barrier which is repeatedly pierced by a cannula. The medical connector may be comprised of as few as three essential parts, each of which are easily formed in mass production. A swabbable elastomeric stopper is also disclosed for closing the open end of a female slip Luer passageway.

### Summary of the Invention

The present invention accordingly provides a medical connector having a longitudinal axis, said connector comprising the features recited in claim 1. Preferred embodiments are specified in the dependent claims.

### Brief Description of the Drawings

In the accompanying drawings:

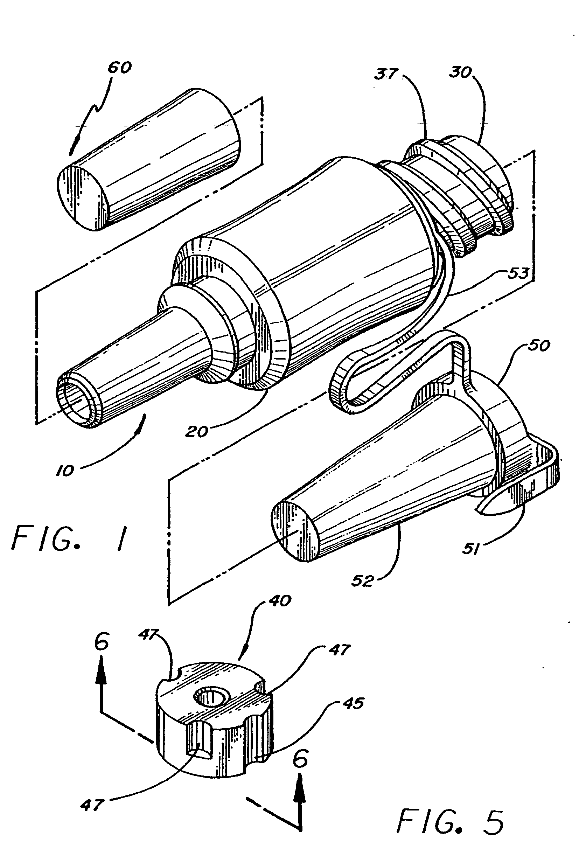

Figure 1 comprises a perspective view of a medical connector according to the present invention including a contamination cap for a male Luer part and a contamination plug/swab for a female Luer part of the connector:
Figure 2 comprises an exploded perspective view of the medical connector of Figure 1;
Figure 3 comprises a longitudinal cross section view showing the medical connector with the valve element in the valve closed position;
Figure 4 is a view like Figure 3 showing the connector in the valve open position;
Figure 5 is a perspective view of the valve element;
Figure 6 is a bottom plan view of the valve element of Fig. 5;
Figure 7 is an end elevation of a valve contacting end of a valve actuator;
Figure 8 is a right end elevation of a valve housing;
Figure 9 is a longitudinal cross section view like Fig. 3 showing a modified housing configuration; and
Figure 10 is a perspective view of a medical connector having the modified housing configuration of Fig. 8.
Figure 11 is a view like Fig. 9 showing an elastomeric safety stopper.
Figures 12a and 12b are perspective views of a first embodiment of the safety stopper shown in Fig. 11.
Figure 13 is a cross-section view at lines 13-13 on Fig. 14 of a second embodiment of safety stopper.
Figure 14 is an end view of the stopper of Fig. 13.

### Description of the Preferred Embodiment

The medical connector 10 of the present invention is essentially a three part connector comprising a valve housing 20 having a male Luer configured end 26 and a female Luer configured valve actuator 30 with a resilient valve element 40 which is compressed between a planar valve seat 22 in the housing 20 and a planar end wall 32 of the valve actuator 30.

The male Luer housing 20 and female Luer valve actuator 30 are aligned on a common longitudinal axis and are rotatable with respect to each other about the longitudinal axis. A concave gripping surface 21 on the housing 20 facilitates fingertip operation of the connector.

The valve housing 20 has a fluid flow passage 24 therein which extends longitudinally from the male Luer fluid line connection end 26 to the valve seat 22. Similarly, the valve actuator 30 has an axially extending female Luer fluid flow passage 34 therein which, in the embodiment shown, terminates in an off-center positioned flow passage 36 in the end wall 32 of the valve actuator which compressively engages the valve element 40. The actuator 30 is molded to have a smooth curved flow transition from axially extending flow passage 34 to off-center passage 36.

The valve element 40 has a fluid flow passageway 44 extending therethrough from an axially aligned opening in fluid communication with passage 24 to an off-center positioned end which can be placed into or out of flow communication with off-center actuator passage 36. Valve element 40 is made of a firm but compressible elastomer which is compressed between the valve seat 22 in the housing and the end wall 32 of the actuator during assembly of the valve. Axial compression of the elastomeric valve element 40 causes radial expansion of the valve element 40 into fluid tight engagement with the surrounding interior cylindrical wall of the housing 20 to provide a tight seal of the valve in open, closed and intermediate positions.

Restraining means on an interior cylindrical wall 28 proximate the open end of the housing 20 and on an exterior cylindrical wall 38 of the valve actuator in the form of an annular bulge 39 on the actuator and mating receiving groove 29 on the housing are dimensioned such that the valve element 40 is compressed to the appropriate extent as the bulge 39 seats itself in the groove 29 during valve assembly. Axial movement of the valve actuator 30 with respect to the housing 20 is thus prevented during operation of the valve. Preferably, abutting radially extending surfaces on the bulge 39 and groove 29 are provided at the location shown to prevent inadvertent withdrawal of the actuator 30 from the housing 20 after the valve has been assembled. Relative rotation between the housing and actuator is, however, permitted. Preferably, a rigid sleeve 46 is provided in the flow passage 44 in the resilient valve element 40 to minimize radial collapse thereof during axial compression of the valve element as the parts of the medical connector are assembled. Sleeve 46, if provided, also prevents all contact of the elastomeric valve element with fluid flow through the connector thus avoiding any possibility of contamination of the fluid by the elastomer or coagulation of blood or other fluid proximate the elastomer.

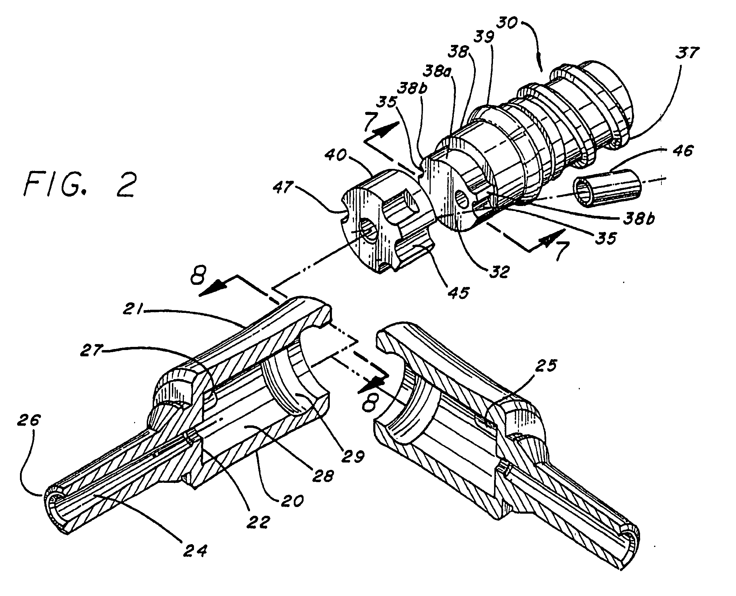

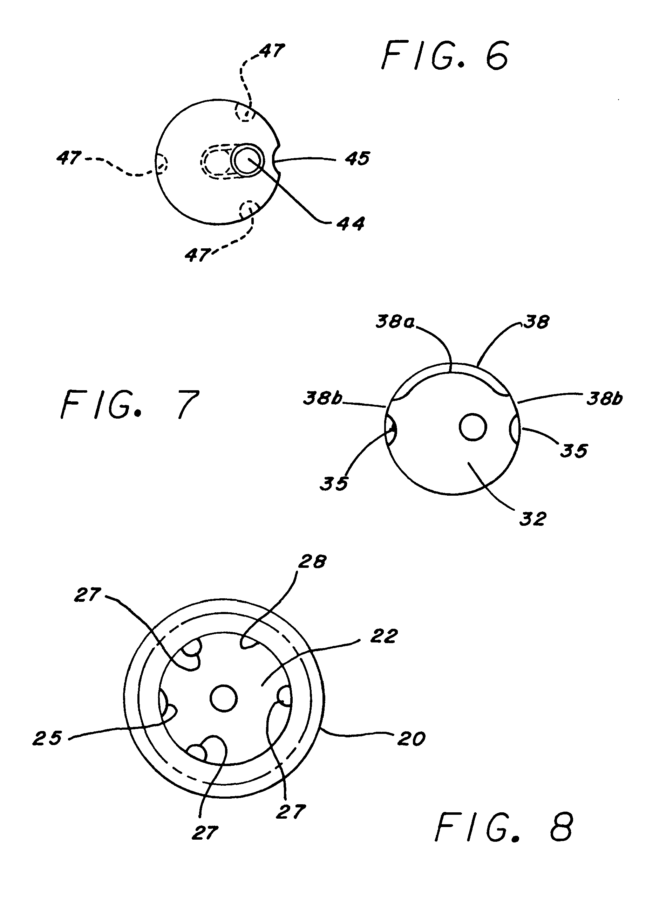

Valve open and valve closed positioning means in the form of a molded long finger 25 on the housing 20 which is received in one or the other of diametrically opposed finger positioning grooves 35 molded in the actuator 30 hold the actuator relative to the housing in either a valve open position or in a valve closed position. The inherent resiliency of the plastic materials of which the housing 20 and valve actuator 30 are made permit the long finger 25 to snap into one or the other of the grooves 35 when the valve is either in the open or closed position while permitting relatively easy manual rotation of the valve actuator 30 relative to the housing 20. The valve element 40 also has a single longitudinally extending groove 45 which receives the long positioning finger 25 on the housing. As seen in Figs. 2 and 7, the exterior cylindrical surface 38 on the actuator 30 extends in excess of 180° proximate the grooves 35 to an annular reduced radius recess 38a of approximately the same depth as grooves 35 to permit the long finger 25 to be received in the annular gap between reduced radius recess 38a and interior housing wall 28 as the actuator 30 is rotated clockwise relative to the housing (as viewed from the actuator end) to open the fluid connection. Radially extending protuberances 38b between the grooves 35 and the recess 38a engage the finger 25 but permit movement of the finger 25 over the protuberances 38b as the connector parts are relatively rotated to open or close the valve so as to provide the user with a "feel" to determine if the valve is in its open or closed position. The full radius portion of surface 38 which extends in excess of 180° prevents further clockwise rotation of the actuator 30 relative to the housing by interference with long finger 25 and requires counterclockwise movement of the actuator relative to the housing to close the fluid connection.

Relative rotation between the valve element 40 and the housing 20 is prevented at all times by mating short fingers 27 molded on the housing which are receivable in short longitudinally extending grooves 47 molded in the valve element. Although three short fingers 27 and grooves 47 are provided to prevent relative rotation between the valve element and the housing 20, as will be apparent, other configurations of mating projections and recesses or adhesive could be used instead to affix the valve element 40 to the valve seat 22 in housing 20. The length of the short fingers 27 is less than the longitudinal length of the grooves 47 on the valve element 40 to freely permit compression of the valve element 40 during assembly without interference between the ends of the fingers 27 and the ends of the grooves 47.

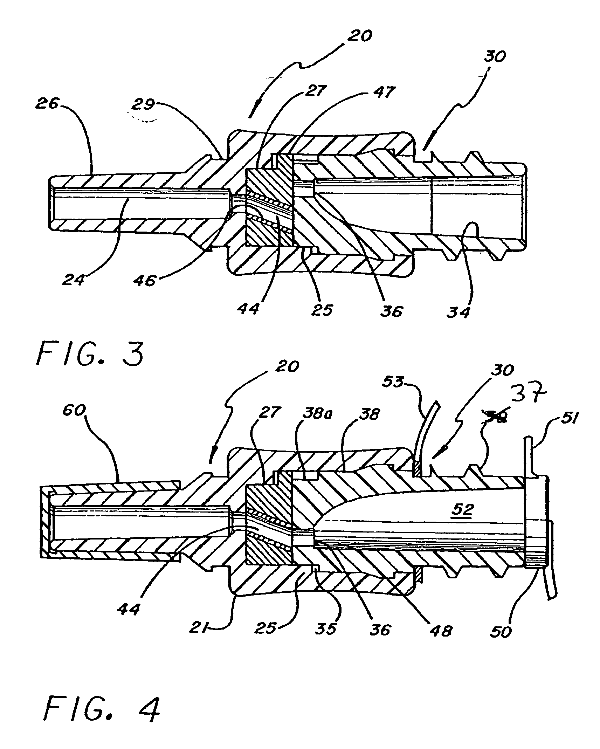

As best seen in Figures 3 and 4, the off-center end of the flow passageway 44 through the valve element 40 is positionable in either a valve closed position (Figure 3) or a valve open position (Figure 4) upon rotation of the valve actuator 30 through an angle of somewhat less than 180° relative to the housing 20.

As shown, the valve actuator 30 has a female Luer tapered receptacle with external threads 37 thereon and the housing 20 has a male tapered Luer flowline connection end 26 provided with a restraining groove 29 for receiving an annular boss on a conventional internally threaded fluid connector line swivel (not shown) which permits threaded connections to a flow line to be made or broken without twisting of the flowline. Preferably, the actuator 30 and the housing 20 are formed of medical grade polycarbonate and the valve element 40 is preferably formed of medical grade polyisoprene.

Either or both of a removable plastic plug 50 which also fulfills the functions of a cleansing swab, and a male end Luer contamination cap 60 may optionally be provided for sealing opposite ends of the medical connector during shipment or otherwise. Plug 50 has a finger grip tab 51 and a male tapered end 52 of variable diameter and length which is formed of a soft plastic and is snugly received in tapered female Luer passageway 34 such that plug 50 may be manually rotated during insertion into passage 34 to swab the passageway all the way to opening to passage 36 with alcohol after disconnection of actuator 30 from an associated flowline. Finger tab 51 permits the plug 50 to be gripped without contamination of the tapered end 52. A plastic tie loop 53 integrally formed with the plug 50 removably affixes the plug to the actuator 30. It will be noted that disconnection of the connector requires counterclockwise rotation of the female threaded line receptacle relative to the actuator 30. The connector is configured such that counterclockwise rotation first causes the actuator 30 to move to the valve closed position before further counterclockwise rotation removes the flowline connector from the conventional clockwise threads 39. Alternatively, the flowline connections can be made or broken by longitudinal insertion or removal of male or female flowline Luer connectors from the female passageway 34 or the male end 26.

The medical connector described and claimed herein is particularly advantageous in that it does not involve the use of any sharp pointed objects such as needles or cannulas. The longitudinally elongated configuration of the connector makes it particularly useful as a replacement for stopcock connectors on a manifold because the radially protruding handle of a stopcock frequently is positioned too close to an adjoining stopcock handle for easy manipulation without interference.

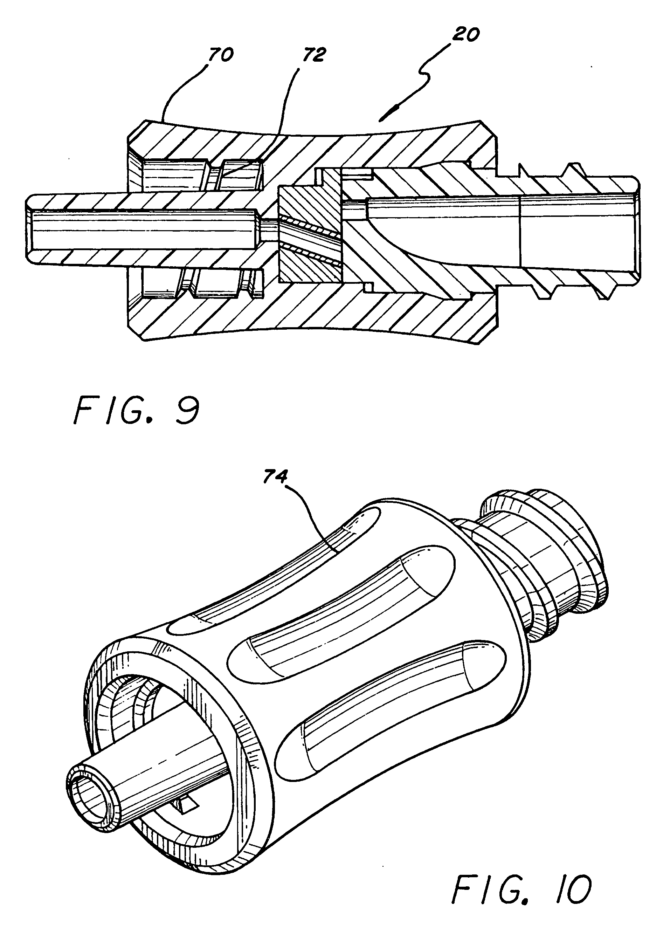

Figures 9 and 10 show a modified housing 20 having an integrally formed internally threaded skirt portion 70 provided with internal threads 72 for receiving a flowline coupling to form a male Luer lock. Housing 20 may also be provided with longitudinally extending non-slip grooves 74 on the exterior of the concave finger gripping exterior surface.

Figures Il and 12 show an optional elastomeric safety stopper 80 comprising a generally cylindrical collar 82 and an integrally formed conical tapered skirt 84. The stopper 80 also has a slitted swabbable outer flap 86 at its outer end and a slitted inner flap 88 closing the inner end of the conical tapered skirt 84. Stopper 80 is positioned, preferably by slight stretching, over the open female Luer end of the valve actuator 30 and is permanently adhesively bonded thereto. A peripheral barb 90 may be formed on the end of the valve actuator 30 to further assist in preventing removal of the safety stopper 80 from the end of the valve actuator 30.

The stopper 80 provides an extra safety feature to prevent inadvertent backflow of blood or other bodily fluid from a patient when a male slip Luer connector end of an IV or other fluid flow line is withdrawn from the female Luer flow passage 34. As discussed above, when disconnecting a threaded Leuer lock flowline, the connector is designed to require automatic closure of the valve to prevent escape of any backflow of fluid from the patient. However, when a slip Luer connector instead of a threaded Luer lock is used on the fluid supply line, it is sometimes possible for a disconnection to be made without first rotating the connector to the valve closed position. The safety stopper 80 is designed to receive a male slip Luer connector through the slitted flap 86 with the four pie shaped flap segments bending inwardly as the male Luer connector is inserted to rest against the inside wall of the female Luer passage 34 in the valve actuator. Although four separate segments are shown in each of the flaps 86, 88, the actual number will be determined by the product designer.

A tapered annular clearance space (not shown) may also be provided in the inside wall of passage 34 to receive and contain the segments of the flap 86 when the male slip Luer is inserted. The thickness of the clearance space may be designed to accommodate the combined wall thickness of the skirt 84 and segments of the flap 86 so that the interior cross-section of the female Luer passage 34 is substantially the same as that of a conventional standard female Luer passage not provided with a safety stopper.

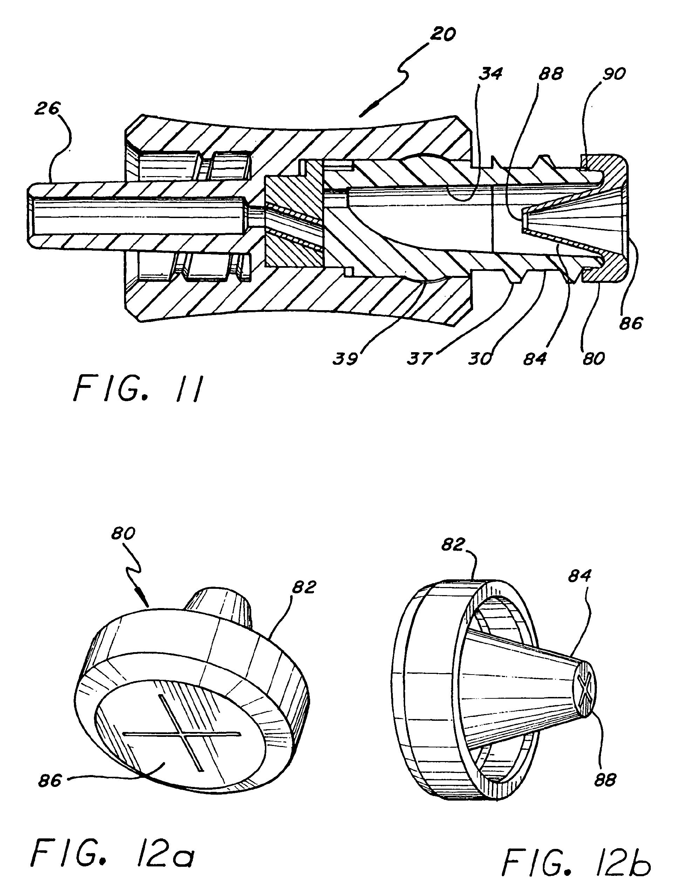

Flow of IV or other fluid to the patient is permitted through the slitted inner flap 88 in the left to right direction as seen in Fig. 11 due to pressure differential established during supply of fluid to the patient but is prevented in the reverse direction when the fluid line connection is broken even if the valve inadvertently remains open. Backflow is doubly prevented by automatic substantially full closure of the inner flap 88 and by substantially full closure of the outer flap 86 (when the male Luer connector is removed) resulting from inherent resiliency of the stopper material. The stopper 80 may be made of any suitable elastomer such as polyisoprene or silicone rubber which is beneficial because it is also inherently lubricous. In addition, the substantially flat exterior surface of the outer flap 86 is easily cleaned or disinfected with a conventional swab which need not enter into the female Luer passage 34 where inadvertently deposited swab material is not easily seen and may not be removed.

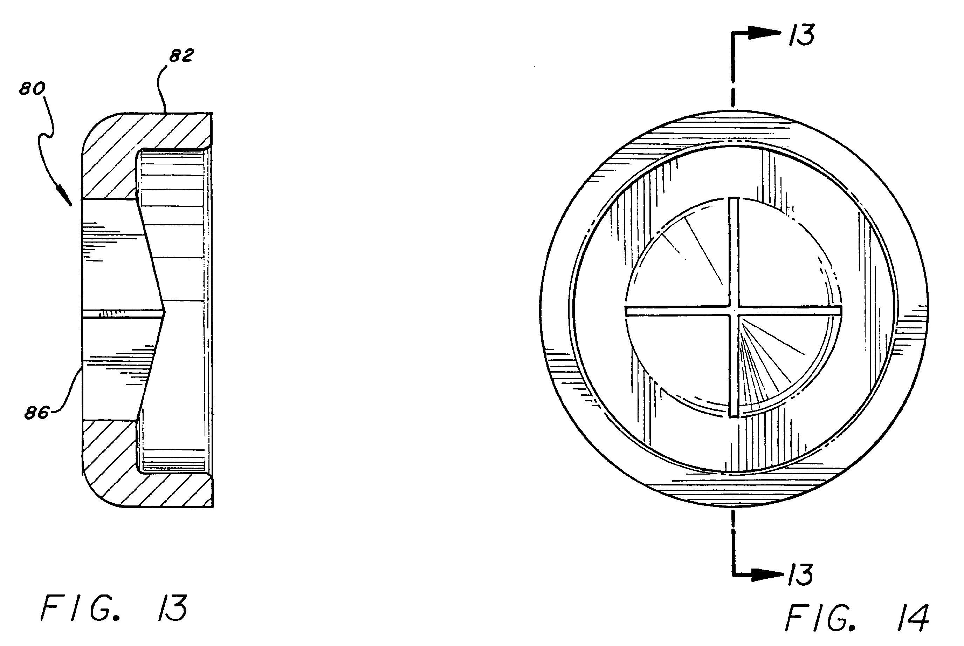

Figs. 13 and 14 show a modified stopper 80 which does not have the tapered skirt 84 and the extra backflow seal provided thereby but which otherwise operates substantially the same as the stopper shown in Figs. 12a and 12b. The stopper 80 of Figs. 13 and 14 has a slightly thickened or conical flap 86 comprised of four segments which, when folded against the inner wall of passageway 34 by insertion of the male Luer connector, form a taper angle which is substantially the same as the taper angle of the male Luer connector. In each of the embodiments of the stopper shown preferably the total length of each of the crossed slits in the outer flap 86 is about 4.06 mm (0.160") which is slightly less than the maximum diameter of a standard male Luer connector 4.29 mm (0.169").

While the foregoing constitutes a complete description of the preferred embodiment, it will be appreciated by persons skilled in the art that modifications can be made from the preferred embodiment and the scope of protection is to be evaluated solely with respect to the attached claims. For example, the valve element 40 may instead be non-rotatably affixed to the end of the valve actuator 30 and the eccentric end 45 of the flow passage through the valve element can face the housing 20 rather than face the actuator 30.

## Claims

1. A medical connector having a longitudinal axis, said connector comprising:
a) a valve housing (20) having a fluid flow passage (24) therein aligned with said longitudinal axis, a valve seat (22) therein, an open end for receiving a compressible valve element (40) and a connection end (26) for connecting said connector to a fluid flow line aligned with said longitudinal axis;
b) a valve actuator (30) having a fluid flow passage (34) therein aligned with said longitudinal axis of said connector, and a valve compressing end wall (32), said fluid flow passage (34) extending through said end wall (32) and ending in an off-center positioned end,
c) a resilient valve element (40) compressed between said valve seat (22) and said valve compressing end wall (32) of said actuator, said valve element having a fluid flow passage (44) therethrough,
d) means (27, 47) connecting said valve element to said housing to prevent relative rotation between said valve element (40) and said housing (20); and
e) restraining means (29, 39) on said housing and on said achiator for restraining relative axial movement therebetween while permitting relative rotation therebetween;
**characterized in that** said valve actuator is mounted in said open end of said housing (20) for rotation about said longitudinal axis with respect to said housings; said flow passage (44) in said valve element extends from an axially aligned opening on a first side of said valve element straight through said valve element to an off-center positioned opening on an opposite side of said valve element; said off-center opening of said fluid passage (44) in said valve element (40) being aligned with said off-center positioned end of said flow passage (34) in said valve actuator when said actuator is rotated relative to said housing to open said valve and said off-center positioned end of said passage (44) in said valve element being circumferentially displaced from said off-center end of said flow passage (34) in said valve actuator when said actuator is rotated relative to said housing to close the valve; and positioning means (25, 35) on said housing and said actuator are provided to hold said actuator in either a valve open position or in a valve closed position relative to said housing.

2. The medical connector of claim 1, **characterized in that** said valve seat (22) in said valve (20) housing has an opening therein axially aligned with said axially positioned opening of said fluid passage (44) in said valve element (40).

3. The medical connector of claim 2, **characterized in that** said connecting means for preventing relative rotation comprises interengaging fingers (27) and grooves (47) on said valve housing (20) and said valve element (40).

4. The medical connector of claim 3, **characterized in that** said fingers (27) are axially extending fingers on said valve housing and said grooves (47) are axially extending grooves on said valve element, said grooves having an axial length less than the axial thickness of said valve element and said fingers having a length less than the axial length of said grooves to permit compression of said valve element during assembly.

5. The medical connector of any one of claims 1 through 4, **characterized in that** said off-center positioned end of the flow passage of said valve actuator (30) is provided as an off-center positioned opening in the end wall 32.

6. The medical connector of any one of the preceding claims, **characterized in that** said positioning means (25, 35) comprises an axially extending finger (25) on a cylindrical interior wall (28) of said valve housing and two axially extending grooves (35) on a cylindrical exterior wall (38) of said valve actuator proximate said valve compressing end.

7. The medical connector of claim 6, **characterized in that** said grooves (35) on said cylindrical wall (38) of said valve actuator are spaced less than 180° from each other and further comprising an axially extending recess (38a) in said cylindrical wall (38) of said valve actuator between said grooves, said recess having approximately the same depth as the depth of said grooves, and radially extending protuberances (38b) between said grooves and said recess.

8. The medical connector of any one of the preceding claims, **characterized in that** said restraining means (28, 39) comprises an elastically engaging annular bulge (39) on an external cylindrical wall of said valve actuator and a bulge receiving groove (39) on an interior cylindrical wall (28) of said valve housing.

9. The medical connector of claim 8, **characterized in that** said bulge and said bulge receiving groove each have abutting radially extending annular surfaces (29a) to maintain axial compression of said valve element and to prevent axial disengagement of said valve housing and said valve actuator.

10. The medical connector of claim 9, **characterized in that** said valve element is axially compressed and radially expanded into sealing engagement with interior walls of said housing during assembly of the connector.

11. The medical connector of claim 10, further **characterized by** a rigid sleeve (46) in said flow passage (44) in said valve element to prevent reduction of the flow cross section during compression of the valve element.

12. The medical connector of any one of the preceding claims, further **characterized by** a concave circular cylindrical exterior finger gripping surface (21) on said housing.

13. The medical connector of any one of the preceding claims, **characterized in that** said valve housing includes a male Luer flowline connector thereon.

14. The medical connector of claim 13, **characterized in that** said valve actuator further comprises a female Luer flowline connector.

15. The medical connector of claim 14, further **characterized by** an external flowline connector thread (37) on said female Luer flowline connector opposite said valve compressing end wall (32) to form a female Luer lock connector.

16. The medical connector of claim 13, 14 or 15, **characterized in that** said male configured Luer end (26) has a retaining groove (29) thereon for receiving and retaining a fluid flow line swivel connector.

17. The medical connector of any one of the preceding claims, **characterized in that** said valve actuator and said valve housing are formed of medical grade polycarbonate and said valve element is formed of medical grade polyisoprene.

18. The medical connector of claims 14 or 15, further **characterized by** an elastomeric safety stopper (80) affixed to said valve actuator to cover a flow passage in said valve actuator, said stopper having at least one slitted flap covering said flow passage in said female Luer flowline connector.

19. The medical connector of claim 18, **characterized in that** two elongated slits in said flap intersect a central region of said flap to provide four flap segments between said slits.

20. The medical connector of claim 19, **characterized in that** each of said slits has a total length of about 0.4 cm (0.160").

21. The medical connector of claim 18, 19 or 20, further **characterized by** adhesive affixing said stopper (80) to said valve actuator.

22. The medical connector of claim 18, **characterized in that** said stopper (80) comprises an integrally formed outer slitted flap (86), an annular collar (82) at the periphery of said outer flap, a tapered skirt (84) extending from said collar away from said outer flap and an inner slitted flap (88) closing an end of said skirt, said collar being affixed to said valve actuator.

23. The medical connector of claim 22, further **characterized by** a recess in an inner wall of said flow passage in said valve actuator (30) for accommodating said skirt (84) and folded segments of said outer flap (86) when a male slip Luer flowline connector is connected to said medical connector.

## Patentansprüche

1. Medizinischer Konnektor mit einer Längsachse sowie:
a) einem Ventilgehäuse (20), das einen mit der Längsachse ausgerichteten Strömungsdurchlass (24) und einen Ventilsitz (22) enthält und ein offenes Ende zur Aufnahme eines komprimierbaren Ventilelements (40) und ein Anschlussende (26) aufweist, das mit der Längsachse ausgerichtet ist und an dem der Konnektor mit einer Fluid-Strömungsleitung verbindbar ist;
b) einem Ventil-Betätigungsglied (30), das einen mit der Längsachse des Konnektors ausgerichteten Strömungsdurchlass (34) und eine das Ventil komprimierende Abschlusswand (32) aufweist, wobei der Fluid-Strömungsdurchlass (34) durch die Abschlusswand (32) verläuft und zu einem exzentrisch angeordneten Ende ausläuft;
c) einem federelastischen Ventilelement (40), das zwischen dem Ventilsitz (22) und der Abschlusswand (32) des Betätigungsglieds komprimiert ist und durch das ein Strömungsdurchlass (44) verläuft;
d) Einrichtungen (27, 47), die das Ventilelement mit dem Gehäuse verbinden, um eine Relativdrehung zwischen dem Ventilelement (40) und dem Gehäuse (20) zu verhindern; und
e) Rückhalteeinrichtungen (29, 39) auf dem Gehäuse und auf dem Betätigungsglied, um eine axiale Relativbewegung zwischen ihnen zu hemmen, aber eine Relativdrehung zwischen ihnen zuzulassen;
**dadurch gekennzeichnet, dass**
das Ventil-Betätigungsglied in das offene Ende des Gehäuses (20) und relativ zu diesem um die Längsachse drehbar eingesetzt ist und dass der Strömungsdurchlass (44) im Ventilelement von einer axial ausgerichteten Öffnung auf einer ersten Seite des Ventilelements gradlinig durch dieses hindurch zu einer exzentrisch liegenden Öffnung auf einer gegenüberliegenden Seite des Ventilelements verläuft, wobei sich die exzentrische Öffnung des Fluid-Durchlasses (44) im Ventilelement (40) mit dem exzentrisch angeordneten Ende des Strömungsdurchlasses (34) im Ventil-Betätigungsglied ausrichtet, wenn man das Betätigungsglied relativ zum Gehäuse dreht, um das Ventil zu öffnen, und das exzentrisch angeordnete Ende des Durchlasses (44) im Ventilelement gegenüber dem exzentrischen Ende des Durchlasskanals 34 im Ventil-Betätigungsglied in Umfangsrichtung versetzt wird, wenn man das Betätigungsglied relativ zum Gehäuse dreht, um das Ventil zu schließen, und dass auf dem Gehäuse und dem Betätigungsglied eine Positioniereinrichtung (25, 35) vorgesehen ist, mit der das Betätigungsglied in einer Offen- oder Schließposition des Ventils relativ zum Gehäuse haltbar ist.

2. Medizinischer Konnektor nach Anspruch 1, **dadurch gekennzeichnet, dass** der Ventilsitz (22) im Ventilgehäuse (20) eine Öffnung enthält, die mit der axial angeordneten Öffnung des Fluid-Durchlasses (44) im Ventilelement (40) axial ausgerichtet ist.

3. Medizinischer Konnektor nach Anspruch 2, **dadurch gekennzeichnet, dass** die Verbindungseinrichtung, mit der eine Relativdrehung verhinderbar ist, ineinander greifende Finger (27) und Nuten (47) auf dem Ventilgehäuse (20) und dem Ventilelement (40) aufweist.

4. Medizinischer Konnektor nach Anspruch 3, **dadurch gekennzeichnet, dass** die Finger (27) axial sich erstreckende Finger auf dem Ventilgehäuse und die Nuten (47) axial verlaufende Nuten auf dem Ventilelement sind und dass die Nuten eine geringere axiale Länge als die axiale Dicke des Ventilelements und die Finger eine geringere axiale Länge als die Nuten haben, um ein Komprimieren des Ventilelements bei der Montage zu ermöglichen.

5. Medizinischer Konnektor nach einem der Ansprüche 1 bis 4, **dadurch gekennzeichnet, dass** das exzentrisch angeordnete Ende des Strömungsdurchlasses im Ventil-Betätigungsglied (30) als exzentrisch liegende Öffnung in der Abschlusswand (32) vorgesehen ist.

6. Medizinischer Konnektor nach einem der vorgehenden Ansprüche, **dadurch gekennzeichnet, dass** die Positioniereinrichtung (25, 35) einen axial sich erstreckenden Finger (25) auf einer zylindrischen Innenwand (28) des Ventilgehäuses und zwei axial sich erstreckende Nuten (35) auf einer zylindrischen Außenwand (38) des Ventil-Betätigungselements am komprimierenden Ende aufweist.

7. Medizinischer Konnektor nach Anspruch 6, **dadurch gekennzeichnet, dass** die Nuten (35) in der zylindrischen Wand (38) des Ventil-Betätigungselements weniger als 180° voneinander beabstandet sind und dass weiterhin in der zylindrischen Wand (38) des Ventil-Betätigungsglieds zwischen den Nuten eine axial verlaufende Ausnehmung (38a), die angenähert gleich tief ist wie die Nuten, sowie zwischen den Nuten und der Ausnehmung radial sich erstreckende Vorsprünge (38b) vorgesehen sind.

8. Medizinischer Konnektor nach einem der vorgehenden Ansprüche, **dadurch gekennzeichnet, dass** die Rückhalteeinrichtung (28, 39) einen elastisch eingreifenden ringförmigen Wulst (39) auf einer zylindrischen Außenwand des Ventil-Betätigungsglied und eine den Wulst aufnehmende Nut (39) in einer zylindrischen Innenwand (28) des Ventilgehäuses aufweist.

9. Medizinischer Konnektor nach Anspruch 8, **dadurch gekennzeichnet, dass** der Wulst und die ihn aufnehmende Nut aneinander anliegende, jeweils radial verlaufende Ringflächen (29a) aufweisen, die den axialen Kompressionszustand des Ventilelements erhalten und ein Lösen des Ventilgehäuses vom Ventil-Betätigungselement verhindern.

10. Medizinischer Konnektor nach Anspruch 9, **dadurch** gekenenzeichnet, dass beim Zusammensetzen des Konnektors das Ventilelement axial komprimiert und radial in den dichten Abschluss gegen die Innenwand des Gehäuses expandiert wird.

11. Medizinischer Konnektor nach Anspruch 10, weiterhin **gekennzeichnet durch** eine starre Hülse (46) im Strömungsdurchlass (44) im Ventilelement, um eine Verkleinerung des Strömungsquerschnitts beim Komprimieren des Ventilelements zu verhindern.

12. Medizinischer Konnektor nach einem der vorgehenden Ansprüche, weiterhin **gekennzeichnet durch** eine außen kreisförmig umlaufende konkave Fingergrifffläche (21) auf dem Gehäuse.

13. Medizinischer Konnektor nach einem der vorgehenden Ansprüche, **gekennzeichnet durch** einen Luer-Leitungssteckverbinder auf dem Ventilgehäuse.

14. Medizinischer Konnektor nach Anspruch 13, **dadurch gekennzeichnet, dass** das Ventil-Betätigungsglied weiterhin eine Luer-Steckaufnahme aufweist.

15. Medizinischer Konnektor nach Anspruch 14, weiterhin **gekennzeichnet durch** ein Leitungsanschluss-Außengewinde (37) auf der Luer-Steckaufnahme gegenüber der komprimierenden Abschlusswand (32), um einen Luer-Lock-Steckaufnahme herzustellen.

16. Medizinischer Konnektor nach Anspruch 13, 14 oder 15, **dadurch gekennzeichnet, dass** auf dem Luer-Steckende (26) eine Rückhaltenut (29) angeordnet ist, die eine Leitungs-Drehverbinder aufnehmen und halten kann.

17. Medizinischer Konnektor nach einem der vorgehenden Ansprüche, **dadurch gekennzeichnet, dass** das Ventil-Betätigungsglied und das Ventilgehäuse aus Polycarbonat und das Ventilelement aus Polyisopren jeweils in medizinischer Qualität gefertigt sind.

18. Medizinischer Konnektor nach Anspruch 14 oder 15, **gekennzeichnet durch** einen Elastomer-Sicherheitsstopfen (80), der am Ventil-Betätigungsglied befestigt einen Durchflusskanal in diesem abdeckt und mindestens eine geschlitzte Lasche aufweist, die den Durchlass in der Luer-Leitungssteckaufnahme bedeckt.

19. Medizinischer Konnektor nach Anspruch 18, **dadurch gekennzeichnet, dass** zwei längliche Schlitze in der Lasche sich in deren Mitte schneiden, um zwischen den Schlitzen vier Laschensegmente herzustellen.

20. Medizinischer Konnektor nach Anspruch 19, **gekennzeichnet**, dass jeder der Schlitze eine Gesamtlänge von etwa 0,4 cm (0.160") hat.

21. Medizinischer Konnektor nach Anspruch 18, 19 oder 20, weiterhin **gekennzeichnet durch** Klebstoff, der den Stopfen (80) am Ventil-Betätigungsglied festlegt.

22. Medizinischer Konnektor nach Anspruch 18, **dadurch gekennzeichnet, dass** der Stopfen (80) eine angeformte äußere geschlitzte Lasche (86), einen ringförmig umlaufenden Kragen (82) am Außenrand der äußeren Lasche, eine verjüngte Schürze (84), die vom Kragen her von der äußeren Lasche weg verläuft und eine innere geschlitze Lasche (88) aufweist, die ein Ende der Schürze verschließt, wobei der Kragen am Ventil-Betätigungsglied befestigt ist.

23. Medizinischer Konnektor nach Anspruch 22, weiterhin **gekennzeichnet durch** eine Vertiefung in einer Innenwand des Strömungsdurchlasses im Ventil-Betätigungsglied (30) zur Aufnahme der Schürze (84) und umgefalteter Segmente der äußeren Lasche (86), wenn an den Konnektor ein Luer-Rutschsteckverbinder angesetzt ist.

## Revendications

1. Raccord médical présentant un axe longitudinal, ledit raccord comprenant :
a) un logement de valve (20) présentant à l'intérieur un passage d'écoulement fluide (24) aligné avec ledit axe longitudinal, un siège de valve (22) à l'intérieur, une extrémité ouverte destinée à recevoir un élément de valve compressible (40) et une extrémité de raccordement (26) destinée à relier ledit raccord à un tuyau d'écoulement fluide aligné avec ledit axe longitudinal ;
b) un actionneur de valve (30) présentant à l'intérieur un passage d'écoulement fluide (34) aligné avec ledit axe longitudinal dudit raccord, et une paroi d'extrémité de compression de valve (32), ledit passage d'écoulement fluide (34) s'étendant à travers ladite paroi d'extrémité (32) et se terminant en une extrémité positionnée de manière excentrée ;
c) un élément de valve résilient (40) comprimé entre ledit siège de valve (22) et ladite paroi d'extrémité de compression de valve (32) dudit actionneur, ledit élément de valve présentant un passage d'écoulement de fluide (44) à travers celui-ci,
d) des moyens (27, 47) raccordant ledit élément de valve au dit logement pour empêcher une rotation relative entre ledit élément de valve (40) et ledit logement (20) ; et
e) des moyens de retenue (29, 39) sur ledit logement et sur ledit actionneur destinés à empêcher le mouvement axial relatif entre eux tout en permettant la rotation relative entre eux ;
**caractérisé en ce que** ledit actionneur de valve est monté dans ladite extrémité ouverte dudit logement (20) en vue d'une rotation autour dudit axe longitudinal par rapport au dit logement ; ledit passage d'écoulement (44) dans ledit élément de valve s'étend d'une ouverture alignée axialement sur un premier côté dudit élément de valve à travers ledit élément de valve jusqu'à une ouverture positionnée de manière excentrée sur un côté opposé dudit élément de valve ; ladite ouverture excentrée dudit passage de fluide (44) dans ledit élément de valve (40) étant alignée avec ladite extrémité positionnée de manière excentrée dudit passage d'écoulement (34) dans ledit actionneur de valve lorsque ledit actionneur est mis en rotation par rapport au dit logement pour ouvrir ladite valve, et ladite extrémité positionnée de manière excentrée (44) dans ledit élément de valve étant déplacée de manière circonférentielle de ladite extrémité excentrée dudit passage d'écoulement (34) dans ledit actionneur de valve lorsque ledit actionneur est mis en rotation par rapport au dit logement pour fermer la valve ; et les moyens de positionnement (25, 35) sur ledit logement et ledit actionneur sont prévus pour maintenir ledit actionneur soit dans une position ouverte de valve, soit dans une position fermée de valve par rapport au dit logement.

2. Raccord médical selon la revendication 1, **caractérisé en ce que** ledit siège de valve (22) dans ledit logement de valve (20) présente à l'intérieur une ouverture alignée axialement avec ladite ouverture positionnée axialement dudit passage de fluide (44) dans ledit élément de valve (40).

3. Raccord médical selon la revendication 2, **caractérisé en que** lesdits moyens de raccordement destinés à empêcher la rotation relative comprennent des doigts (27) et rainures (47) se mettant mutuellement en prise sur ledit logement de valve (20) et ledit élément de valve (40).

4. Raccord médical selon la revendication 3, **caractérisé en ce que** lesdits doigts (27) sont des doigts s'étendant axialement sur ledit logement de valve et lesdites rainures (47) sont des rainures s'étendant axialement sur ledit élément de valve, lesdites rainures présentant une longueur axiale inférieure à l'épaisseur axiale dudit élément de valve et lesdits doigts présentant une longueur inférieure à la longueur axiale desdites rainures pour permettre la compression dudit élément de valve pendant l'assemblage.

5. Raccord médical selon l'une quelconque des revendications 1 à 4, **caractérisé en ce que** ladite extrémité positionnée de manière excentrée du passage d'écoulement dudit actionneur de valve (30) est prévue en tant qu'ouverture positionnée de manière excentrée dans la paroi d'extrémité (32).

6. Raccord médical selon l'une quelconque des revendications précédentes, **caractérisé en ce que** lesdits moyens de positionnement (25, 35) comprennent un doigt (25) s'étendant axialement sur une paroi intérieure cylindrique (28) dudit logement de valve et deux rainures (35) s'étendant axialement sur une paroi extérieure cylindrique (38) dudit actionneur de valve situé à proximité de ladite extrémité de compression de valve.

7. Raccord médical selon la revendication 6, **caractérisé en que** lesdites rainures (35) sur ladite paroi cylindrique (38) dudit actionneur de valve sont espacées l'une de l'autre de moins de 180° et comprenant en outre un évidement (38a) s'étendant axialement dans ladite paroi cylindrique (38) dudit actionneur de valve entre lesdites rainures, ledit évidement présentant de manière approximative la même profondeur que la profondeur desdites rainures, et des protubérances (38b) s'étendant radialement entre lesdites rainures et ledit évidement.

8. Raccord médical selon l'une quelconque des revendications précédentes, **caractérisé en ce que** lesdits moyens de retenue (28, 39) comprennent un renflement annulaire (39) se mettant en prise élastiquement sur une paroi cylindrique externe dudit actionneur de valve et une rainure de réception du renflement (39) sur une paroi cylindrique intérieure (28) dudit logement de valve.

9. Raccord médical selon la revendication 8, **caractérisé en que** ledit renflement et ladite rainure de réception du renflement présentent chacun des surfaces annulaires s'étendant radialement en about (29a) pour maintenir la compression axiale dudit élément de valve et pour empêcher le désenclenchement axial dudit logement de valve et dudit actionneur de valve.

10. Raccord médical selon la revendication 9, **caractérisé en que** ledit élément de valve est comprimé axialement et étendu radialement en mise en prise étanche avec les parois intérieures dudit logement lors de l'assemblage du raccord.

11. Raccord médical selon la revendication 10, **caractérisé en outre par** un manchon rigide (46) dans ledit passage d'écoulement (44) dans ledit élément de valve pour empêcher la réduction de la section transversale de l'écoulement pendant la compression de l'élément de valve.

12. Raccord médical selon l'une quelconque des revendications précédentes, **caractérisé en outre par** une surface de préhension digitale extérieure cylindrique et circulaire concave (21) sur ledit logement.

13. Raccord médical selon l'une quelconque des revendications précédentes, **caractérisé en ce que** ledit logement de valve comprend un raccord de tuyau d'écoulement Luer mâle.

14. Raccord médical selon la revendication 13, **caractérisé en que** ledit actionneur de valve comprend en outre un raccord de tuyau d'écoulement Luer femelle.

15. Raccord médical selon la revendication 14, **caractérisé en outre par** un filet externe (37) de raccord de tuyau d'écoulement sur ledit raccord de tuyau d'écoulement Luer femelle opposé à ladite paroi d'extrémité de compression de valve (32) pour former un raccord Luerlock femelle.

16. Raccord médical selon la revendication 13, 14 ou 15, **caractérisé en que** ladite extrémité Luer mâle (26) présente une rainure de retenue (29) destinée à recevoir et à retenir un raccord pivotant de tuyau d'écoulement de fluide.

17. Raccord médical selon l'une quelconque des revendications précédentes, **caractérisé en ce que** ledit actionneur de valve et ledit logement de valve sont fabriqués en polycarbonate de qualité médicale et ledit élément de valve est fabriqué en polyisoprène de qualité médicale.

18. Raccord médical selon la revendication 14 ou 15, **caractérisé en outre par** un bouchon de sécurité élastomère (80) fixé au dit actionneur de valve pour recouvrir un passage d'écoulement dans ledit actionneur de valve, ledit bouchon présentant au moins un volet fendu couvrant ledit passage d'écoulement dans ledit raccord de tuyau d'écoulement Luer femelle.

19. Raccord médical selon la revendication 18, **caractérisé en ce que** deux fentes allongées dans ledit volet croisent une région centrale du dit volet pour former quatre segments de volet entre lesdites fentes.

20. Raccord médical selon la revendication 19, **caractérisé en que** chacune desdites fentes a une longueur d'environ 0,4 cm (0,160 pouce).

21. Raccord médical selon la revendication 18, 19 ou 20, **caractérisé en outre par** un adhésif fixant ledit bouchon (80) au dit actionneur de valve.

22. Raccord médical selon la revendication 18, **caractérisé en que** ledit bouchon (80) comprend un volet fendu externe (86) formé d'un seul tenant, un collier annulaire (82) à la périphérie dudit volet externe, une collerette effilée (84) s'étendant depuis ledit collier en s'éloignant dudit volet externe et un volet fendu interne (88) fermant une extrémité de ladite collerette, ledit collier étant fixé au dit actionneur de valve.

23. Raccord médical selon la revendication 22, **caractérisé en outre par** un évidement dans une paroi interne dudit passage d'écoulement dans ledit actionneur de valve (30) destiné à recevoir ladite collerette (84) et les segments pliés dudit volet externe (86) lorsque un raccord de tuyau d'écoulement Luer mâle à coulissement est relié au dit raccord médical.
